# EUROPEAN PATENT APPLICATION

(11) **EP 2 368 599 A1**
(43) Date of publication of application: **28.09.2011**
(21) Application number: 11158036.1
(22) Date of filing: 14.03.2011
(51) Int. Cl.: A61N 5/06

(54) **Hair growth device**

(30) Priority: 26.03.2010 JP 2010073632
(71) Applicant: Panasonic Electric Works Co., Ltd., Kadoma-shi Osaka 571-8686 (JP)
(72) Inventor: Takada, Kosaku, Kadoma-shi Osaka 571-8686 (JP)
(74) Representative: Appelt, Christian W.

(57) **Abstract**

A hair growth device includes a first light irradiator configured to irradiate an invisible light of invisible wavelength range, for hair growth; and a second light irradiator configured to irradiate a visible light of visible wavelength range. The second light irradiator is configured to direct the visible light to either inside of a light irradiation area of the light from the first light irradiator or outer circumference of the light irradiation area.

## Description

### Technical Field

The present invention relates to a hair growth device.

### Background Art

In the prior art, there is hair growth technology using light, which activates hair growing structures so as to promote the growth of hair, by irradiating (exposing) light of a main irradiation wavelength in a range of 390 nm to 1600 nm onto hair growing structures such as follicles and the periphery thereof such as skin, as in Japanese Patent Application Publication No. 2005-512620, for instance.

However, for users, such as a therapist or patient receiving treatment, the wavelength range from 360 nm to 780 nm is generally a visible range. Therefore, when light in a wavelength range outside the visible range is used as light for hair growth in a conventional hair growth device, the user is not able to see the emission of light by the light source, and it is not possible to verify that the light for hair growth is being irradiated correctly just by means of an operating lamp on an operating panel, for example.

Because it is not possible to verify that the light for hair growth is being irradiated correctly, then there are problems in that the user may mistakenly identify a fault or feel insecure about using the device. It is possible to judge that light for hair growth is being irradiated correctly by, for example, observing that the light source is emitting light (that irradiated light is being output), or that light is striking a part (irradiation object) where it is wished to impart a hair growing effect (that the irradiation object is in the light irradiation area where light is irradiated), or the like.

Since it is difficult for a user to perceive a hair growing effect with short-term use, such as after one light irradiation operation, then there is a risk that a feeling of insecurity will lead to reduced use of the hair growth device, and hence there is a problem in that it is difficult to achieve a stable hair growing effect in the user.

### Summary of Invention

In view of this situation, the invention aims to provide an optical hair growth device whereby a user can judge readily whether or not light for hair growth is being irradiated correctly.

In order to resolve this problem, the hair growth device according to the present invention comprises a first light irradiator configured to irradiate an invisible light of invisible wavelength range, for hair growth; and a second light irradiator configured to irradiate a visible light of visible wavelength range. For example, the invisible light is directed to an area on a user's skin. The second light irradiator is configured to direct the visible light to either inside of a light irradiation area of the light from the first light irradiator or outer circumference of the light irradiation area.

Furthermore, it is preferred that a main irradiation wavelength of the light irradiated from the first light irradiator is included in a wavelength range between 780 nm and 2000 nm.

Moreover, it is preferred that the hair growth device further comprises a controller configured to vary at least one of brightness and color of the light irradiated from the second light irradiator in response to an irradiation time of the light irradiated from the first light irradiator.

Furthermore, it is preferred that the hair growth device further include a controller configured to change at least one of brightness and color of the visible light when the first light irradiator stops irradiating the invisible light.

Moreover, it is preferred that the first light irradiator comprises a plurality of radiation fronts from each of which the invisible light is irradiated, while the second light irradiator comprises a plurality of radiation fronts from each of which the visible light is irradiated, wherein the plurality of the radiation fronts of the first light irradiator are arranged in an alternating relation to the plurality of the radiation fronts of the second light.

Furthermore, it is preferred that the second light irradiator comprises a plurality of radiation fronts arranged in an annular shape.

Moreover, it is preferred that the first light irradiator comprises a plurality of radiation fronts arranged in a semi-spherical shape, each of light irradiation directions from the radiation fronts being arranged so as to face towards the center of the semi-spherical shape.

By adopting a composition of this kind, it is possible for a user readily to judge, by means of light irradiated from a second light source, whether light for hair growth, which is light irradiated from a first light source, is being irradiated correctly.

### Brief Description of Drawings

Preferred embodiments of the invention will now be described in further details. Other features and advantages of the present invention will become better understood with regard to the following detailed description and accompanying drawings where:
Fig. 1 is a schematic drawing for illustrating respective irradiating units of one example of an embodiment of the invention;
Fig. 2 is an enlarged diagram of the principal part of same;
Figs. 3A and 3B are plan diagrams of examples where irradiating units of a first irradiating unit and a second irradiating unit are arranged alternately;
Figs. 4A and 4B are plan diagrams of examples where irradiating units of a second irradiating unit are arranged at the outer circumference of a first irradiating unit;
Fig. 5 is a plan diagram of a modification example of Fig. 4; and
Fig. 6 is a side diagram showing a partial cross- unit of an example where radiation fronts are arranged in a semi-spherical shape.

### Description of Embodiments

Below, one example of an embodiment is described on the basis of the drawings.

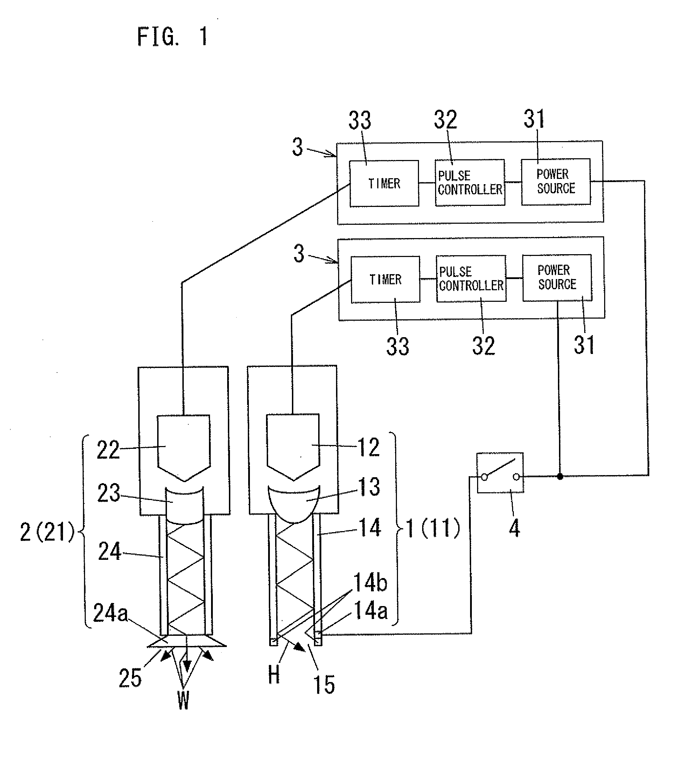

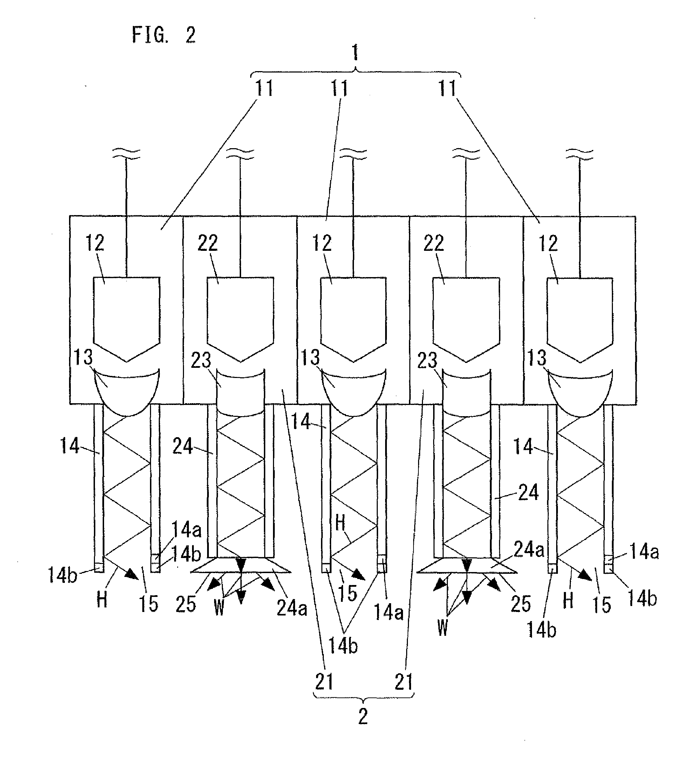

As shown in Figs. 1 and 2, the hair growth device according to the present embodiment comprises a first irradiating unit 1 (first light irradiator) configured to irradiate light of an invisible wavelength range, a second irradiating unit 2 (second light irradiator) configured to irradiate light of a visible wavelength range, and control boxes 3 (controller) configured to control irradiation of the respective irradiating units. This device is positioned in contact with (abutting against) or in the proximity of an irradiation object 6, namely, hair growth structures and the periphery thereof, or the like, and light emitted from the first irradiating unit 1 and the second irradiating unit 2 is directed onto the irradiation object 6. The light irradiated from the first irradiating unit 1 is light which raises the hair growing effect of promoting the growth of body hair and head hair, and the like, on the irradiation object 6.

In the present embodiment, the visible wavelength range has a main irradiation wavelength (peak wavelength) at about 380 nm to 780 nm, and the invisible wavelength range has a main irradiation wavelength at 780 nm or above, which is longer than the wavelength of the visible range. Desirably, the invisible wavelength range is a near-infrared wavelength range having a main irradiation wavelength at 780 nm to 2000 nm. Moreover, in a near-infrared wavelength range, it is desirable to use a specific absorption wavelength of water, such as the vicinity of 950 nm, the vicinity of 1150 nm, the vicinity of 1450 nm, the vicinity of 1790 nm, or the like, as the main irradiation wavelength, so as to perform light irradiation which is not liable to cause inflammation of the irradiation object 6. The specific absorption wavelength of the water is a wavelength which shows stronger light absorbance than other wavelengths, or shows sudden change in light absorbance, when irradiated on water, and is caused by the O-H linking groups in the water.

The first irradiating unit 1 includes a plurality of irradiating units 11 each of which is formed of a first light source 12 configured to emit (invisible) light of an invisible wavelength range, a light guide unit 14 configured to guide the light from the first light source 12, and a condensing lens 13 which is arranged between the first light source 12 and the light guide unit 14 and is configured to condense the light from the first light source 12 in the light guide unit 14.

The first light source 12 comprises a light-emitting diode (LED) as a light source member, for example. The light guide unit 14 is a round cylindrical member having, on the inner circumference thereof, a reflection front (plane) made of a metal material such as a gold, silver or aluminum, one end (a first end) of the cylinder being oriented towards the first light source 12 and the other end (a second end) thereof being oriented towards the exterior.

In other words, the irradiating unit 11 collects light from the first light source 12 on first end side of the light guide unit 14 by means of the condensing lens 13, and radiates this light externally via the light guide unit 14, thereby irradiating light of an invisible wavelength range which is light for hair growth on the irradiation object 6.

The reference numeral 14a on the second end of the light guide unit 14 indicates an irradiation object detecting unit, such as a pressure sensor, temperature sensor or moisture detection sensor, or the like, which detects that an irradiation object 6 has contacted or abutted against the second end of the light guide unit 14. The reference numeral 14b is an attachment, such as an elastic body, which protects against damage or breakage when the second end of the light guide unit 14 or the irradiation object detection unit contacts the irradiation object 6, or the like. Moreover, reference numeral 4 is an on/off switch which enables irradiation of light from the respective irradiating units by switching on, for instance, with a detection signal output when an irradiation object 6 is detected by the irradiation object detecting unit 14a, and this switch forms a safety management device which manages the safety of light irradiation in relation to the user.

Furthermore, the second irradiating unit 2 has substantially the same composition as the first irradiating unit 1, and includes a plurality of irradiating units 21 each of which is formed of a second light source 22, a condensing lens 23 and a light guide unit 24. A light expanding unit 24a, such as an expanding lens, is attached to the second end of the light guide unit 24, instead of the irradiation object detecting unit 14a and the attachment 14b.

Desirably, the light guide units 24 of the second irradiating unit 2 are shorter than the light guide units 14 of the first irradiating unit 1 in the axial direction by an amount equivalent to the light expanding units 24a, and the attachments 14b of the first irradiating unit 1 are provided so as to contact the irradiation object 6 before the light expanding units 24a. Below, light irradiated from the first irradiating unit 1 is called "hair growth light H" and light irradiated from the second irradiating unit 2 is called "visible light W".

In this way, the radiation fronts (planes) 15 from which the first irradiating unit 1 radiates hair growth light H externally are the second end openings of the light guide units 14, and the radiation fronts (planes) 25 from which the second irradiation unit 2 radiates visible light W externally are the front ends of the light expanding units 24a installed on the second ends of the light guide units 24. Consequently, the light irradiation area in which hair growth light H from the first irradiating unit 1 is irradiated onto an irradiation object 6 abutted thereagainst, or the like, has a circular shape of substantially the same diameter as the open end faces of the light guide units 14 which constitute the radiation fronts 15, and the light irradiation area of the second irradiating unit 2 is a circular shape of larger diameter than the radiation fronts 25, due to the light expanding units 24a.

The radiation fronts 15 and 25 and the light irradiation areas are not limited to circular shapes, and may also have a rectangular or elliptical shape, being set appropriately in accordance with the shapes of the condensing lenses 13, 23, the light guide units 14, 24, and the light expanding units 24a, and the like. What is more, the radiation fronts 25 of the second irradiating unit 2 may have a circular arc shape which follows the outer circumferences of the radiation fronts 15 of the first irradiating unit 1, or a fan shape, or the like, which covers the gaps occurring in an arrangement of a plurality of radiation fronts 15 of the first irradiating unit 1.

Furthermore, the first irradiating unit 1 and the second irradiating unit 2 are provided inside a round cylindrical irradiation case 5, and the radiation fronts 15 are arranged in substantially the same plane as a circular open end 5a of the irradiation case 5.

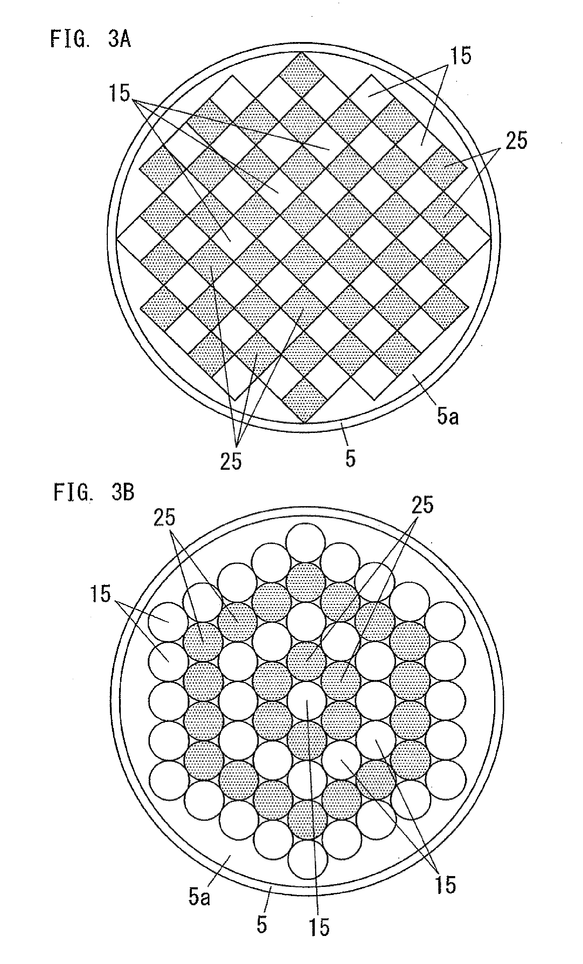

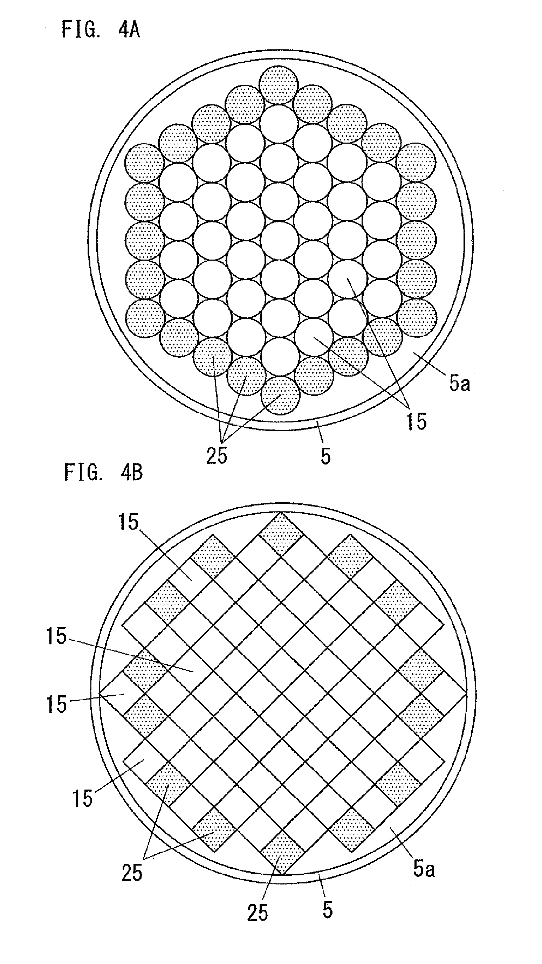

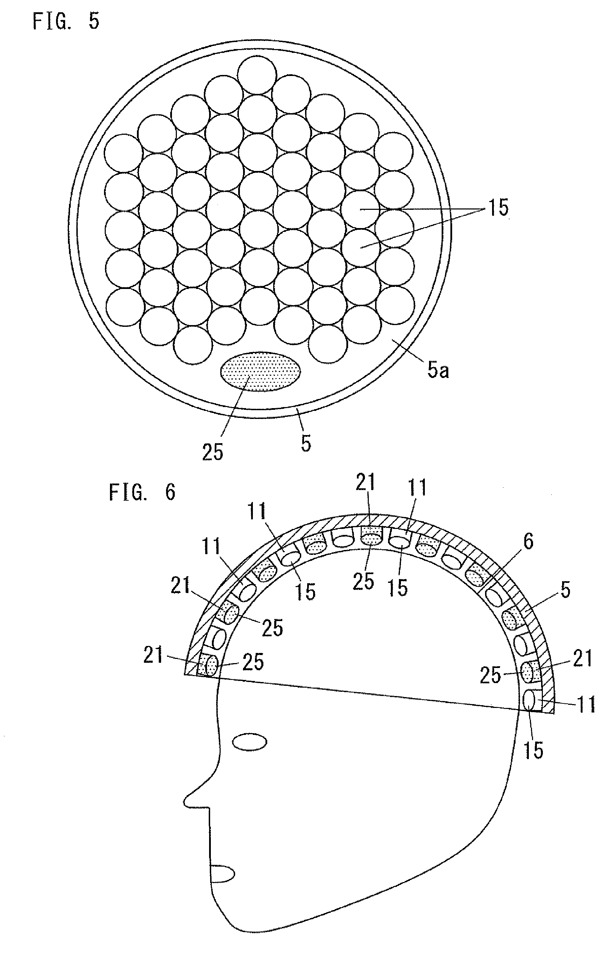

The arrangement of the radiation fronts 15 and 25 of the first irradiating unit 1 and the second irradiation unit 2 may be an alternating arrangement where the respective radiation fronts 15, 25 are mutually alternating in plan view, as shown in Fig. 3, or an arrangement where the second irradiating unit 2 is arranged about the outer circumference of the first irradiating unit 1, as shown in Figs. 4 and 5.

More specifically, in the arrangement shown in Fig. 3A, a plurality of radiation fronts 15, 25 of the respective irradiating units 11, 21 are arranged respectively on substantially straight lines in each irradiating unit, and the radiation fronts 15 aligned linearly in the first irradiating unit 1 and the radiation fronts 25 aligned linearly in the second irradiating unit 2 are arranged in alternating fashion. In other words, the radiation fronts 15 of the first irradiating unit 1 and the radiation fronts 25 of the second irradiating unit 2 are arranged in an alternating matrix configuration.

The arrangement shown in Fig. 3B is one where the radiation fronts 15, 25 of respective irradiating units 11, 21 are arranged in annular shapes having different diameters, in each irradiating unit, and the radiation fronts 15 and 25 arranged in an annular shape are disposed in alternating fashion from a radiation front 15 of one irradiating unit 11 of the first irradiating unit 1 which is positioned at the center.

By arranging the first irradiating unit 1 and the second irradiating unit 2 alternately in this way, the light irradiation areas of the second irradiating unit 2 are disposed between the light irradiation areas of the first irradiating unit 1, and furthermore a portion of the light irradiation areas of the second irradiating unit 2 overlaps with the light irradiation areas of the first irradiating unit 1.

Therefore, visible light (W) is present in the light irradiation area of the hair growth light H and the periphery thereof, and hence it is possible to recognize the presence or absence of a light irradiation operation, even if the user does not check an indicator unit, such as an operating lamp, or the like, which is provided in an operating unit (not illustrated in particular) of the hair growth device.

In other words, the operation of the device, for instance, whether irradiation light is being emitted, and the irradiation position, for instance, whether the irradiation light is striking the irradiation object 6, can be confirmed easily from the presence or absence of visible light W on the irradiation object 6, and the user is not liable to feel a sense of insecurity. Consequently, it is possible to encourage regular repeated use (continued use) of the hair growth device by a user, and a hair growing effect can be imparted readily and stably.

The visible light W from the radiation fronts 25 of the second irradiating unit 2 positioned on the outer circumference side is radiated outwards as reflected light from the surface and interior of the irradiation object 6, even if the radiation fronts 25 are in contact with the irradiation object 6, or the like, and therefore the visible light W is readily perceived by the user. Moreover, since the radiation fronts are arranged alternately, the user readily obtains a visual impression of the hair growth light H striking the irradiation object 6, and furthermore blood flow in the periphery of the hair growing structure is promoted by the heat from the visible light W, thus making it possible to achieve a hair growing effect more readily.

Furthermore, the arrangement shown in Fig. 4A is one where the radiation fronts 15 of the irradiating units 11 of the first irradiating unit 1 are arranged in a circular shape and the radiation fronts 25 of the second irradiating unit 2 are arranged in an annular shape having a larger diameter than the circular shape, in such a manner that the radiation fronts 25 of the second irradiating unit 2 surround the periphery of the radiation fronts 15 of the first irradiating unit 1. The arrangement shown in Fig. 4B is one where radiation fronts 15 of the first irradiating unit 1 are arranged in a rectangular shape, and a portion of the radiation fronts 15 in the outer circumference are substituted with radiation fronts 25 of the second irradiating unit 2, spaced at prescribed intervals apart.

In this way, by arranging a second irradiating unit 2 on the outer side of the first irradiation unit 1, the light irradiation area of the first irradiating unit 1 is surrounded wholly or partially by the light irradiation areas of the second irradiating unit 2, and furthermore a portion of the light irradiation areas of the second irradiating unit 2 overlaps with the light irradiation area of the first irradiating unit 1. Therefore, visible light (W) is present in the periphery of the light irradiation area of the hair growth light H, and hence it is possible to recognize the presence or absence of a light irradiation operation, even if the user does not check a peripheral indicator unit in an operating unit, or the like, of the hair growth device.

In other words, the operation of the device, for instance, whether irradiation light is being emitted, and the irradiation position, for instance, whether the irradiation light is striking the irradiation object 6, can be confirmed easily from the presence or absence of visible light W on the irradiation object 6, and the user is not liable to feel a sense of insecurity.

Since the radiation fronts 25 of the second irradiating unit 2 are positioned on the outer circumference of the first irradiating unit 1, then visible light W is radiated outwards as reflected light from the surface and interior of the irradiation object 6, even if the radiation fronts 25 are in contact with the irradiation object 6, or the like, and therefore the visible light W is readily perceived by the user. Moreover, since the visible light W is positioned so as to follow the outer perimeter of the irradiation area of the hair growth light H, it is possible to make the effective range in which a hair growing effect is obtained more readily visible to the user.

Furthermore, the arrangement shown in Fig. 5 is one where radiation fronts 15 of a first irradiating unit 1 are arranged in a circular shape, an empty portion where radiation fronts 15 of the first irradiating unit 1 are not provided is formed at one location on the outer circumference of the circle, and one radiation front 25 of a second irradiating unit 2 is arranged in this empty portion. The visible light W of the second irradiating unit 2 strikes a position on the irradiation object 6 which opposes the empty portion, and the light irradiation area of the second irradiating unit 2 does not overlap with the light irradiation area of the first irradiating unit 1, but is positioned at the outer circumference of the light irradiation area of the first irradiating unit 1.

Consequently, visible light (W) is present in the vicinity of the light irradiation area of the hair growth light H, the user is able readily to verify the operation of the device and to judge the irradiation position from the presence or absence of visible light W on the irradiation object 6, and the user is not liable to feel a sense of insecurity. Since the radiation fronts 25 of the second irradiating unit 2 are positioned on the outer circumference of the first irradiating unit 1, then visible light W is radiated outwards as reflected light from the surface and interior of the irradiation object 6, even if the radiation fronts 25 are in contact with the irradiation object 6, or the like, and therefore the visible light W is readily perceived by the user.

Furthermore, the control boxes 3 for controlling the irradiating units are provided for the first irradiating unit 1 and the second irradiating unit 2, each of which comprises a power source unit 31 for supplying electrical power to a corresponding irradiating unit, a pulse controller 32 configured to cause the light source to pulse on and off, and a timer 33 configured to count the light irradiation time. The control box 3 of the present invention is not limited to the control boxes for the first irradiating unit 1 and the second irradiating unit 2, and it is also possible for the control box 3 to share use of the power supply unit 31, pulse controller 32 and timer 33, or to provide a timer 33 in only one portion.

The pulse controller 32 pulses the light sources on and off, and also serves as a controller for light adjustment (dimming) which implements control for varying the brightness and color (wavelength) of the irradiated light from the radiation fronts 15 and 25 of the respectively corresponding irradiating units.

For example, the pulse controller 32 for the first irradiating unit 1 may, for example, control and change the wavelength of the irradiated light from the first irradiating unit 1 so as to correspond to the amount of moisture in the irradiation object 6, and the like, by switching between the vicinity of 950 nm and the vicinity of 1450 nm, which is more readily absorbed by water.

In this case, for example, the first light sources 12 include a light source member having a main irradiation wavelength in the vicinity of 950 nm and a light source member having a main irradiation wavelength in the vicinity of 1450 nm, and the pulse controller 32 switches the light source member which emits light for introduction into the condensing lens 13 and the light guide unit 14. The composition is not limited to switching the light source members given as an example above, and it is also possible to change the main irradiation wavelength of the hair growth light H from the radiation fronts 15 by limiting a portion of the wavelength range of the light emitted by the light source member, by means of a wavelength selecting filter, or the like. Of course, the pulse controller 32 is not limited to switching wavelengths between the vicinity of 950 nm and the vicinity of 1450 nm.

The brightness of the irradiated light from the first irradiating unit 1 may be controlled, for example, by switching off the first light sources 12 upon reaching a time of 15 minutes or more, which is considered to be an irradiation time during which an enhanced hair growing effect is obtained with the hair growth light H. Changing the brightness of the hair growth light H is not limited to switching off the first light sources 12, and it is also possible to provide apertures, such as shutter members, so as to reduce, or substantially shut off, the amount of hair growth light H radiated from the radiation fronts 15.

Furthermore, in the case of the pulse controller 32 for the second irradiating unit 2, the wavelength of the irradiated light from the second irradiating unit 2 may be controlled and changed by varying the color of the visible light W in accordance with the measurement time of the timer 33, and reporting the light irradiation time by the first irradiating unit 1.

In this case, for example, the second light sources 22 each have a plurality of light source members having different emission colors, and the pulse controller 32 changes the color of the visible light W from the radiation fronts 25 by switching the light source member which emits light, from amongst these light source members, for instance. The time at which the color is changed may be a uniform time interval, or when the irradiation time of the hair growth light H has reached 15 minutes or more, which is the end time of the light irradiation operation, or several minutes before the end time, or the like.

By changing the color of the visible light W in this way, it is possible to inform the user of the elapsed time of light irradiation, the remaining time until the end of light irradiation, or the operational status of the hair growth device, such as the end of irradiation of the hair growth light H. Consequently, a user is able to ascertain readily the operational status of the hair growth device from the color of the light, simply by observing the visible light W which strikes the irradiation object 6, and hence the user can easily recognize the operational status of the device.

The main irradiation wavelength of the visible light W may be changed by restricting a portion of the wavelength range of the light emitted by the light source members, by means of a wavelength selecting filter, or the like. What is more, the visible light W may be controlled and changed by altering the luminosity of the second light sources 22 so as to make the visible light H darker or brighter, by switching from an on state to a flashing state, by changing the flashing period, or by combining change of the color and change of the brightness.

Furthermore, the light source units are not limited to an LED, and may be constituted by a short wavelength laser, or a halogen lamp or broad-band laser in which the wavelength of the irradiated light is restricted to a specific wavelength range by providing a wavelength selecting filter in the irradiating unit, or the like. Of course, it is also possible to use different light source members respectively in the first light sources 12 and the second light sources 22. By using a light source member having a plurality of peak wavelengths in the light emission colors, such as a halogen lamp, for the second light source, it is possible to change the color of the visible light W without providing a plurality of light source members.

Moreover, it is also possible to use a light guide unit which is flexible and can change shape while preserving light guiding performance, such as an optical fiber, and to supply light to a plurality of light guide units from one light source using an expanding lens, or the like, instead of a condensing lens. When a light source is to be shared in this way, it is desirable to make shared use of the second light source 22 of the irradiating units 21 of the second irradiating unit 2 which does not serve for hair growth, since this allows costs to be reduced without lowering the hair growing effect. Furthermore, by forming the casings of the irradiating units 11 and 21 and the members, such as condensing lenses 13, 23, with a common shape and dimensions in the first irradiating unit 1 and the second irradiating unit 2, the costs involved in manufacture and inspection can be reduced.

The shape of the open end 5a of the irradiation case 5 is not limited to a circular shape, and may also be a rectangular shape or an elliptical shape. Of course, the arrangement of the radiation fronts 15 and 25 is not limited to the examples given, and the radiation fronts 15 of the first irradiating unit may be arranged in a circular shape about a centrally positioned radiation front 25 of a second irradiating unit, or may be arranged in a shape which does not follow the open end 5a of the irradiation case 5 (for instance, a rectangular shape with respect to a circular shape). What is more, the second irradiating unit 2 may also serve as an irradiating unit for hair growth, for instance, by changing the irradiated light from the radiation fronts 15 from visible light W to hair growth light H, by varying the wavelength, or the like, when the device is placed in contact, or the like, with the irradiation object 6, and returning the irradiated light to visible light W again when the time period for performing light irradiation (for instance, 15 minutes) has elapsed, or the like.

Furthermore, the radiation fronts 15 and 25 are not limited to a planar arrangement and it is also possible to arrange the radiation fronts in a plate shape which curves in a circular arc shape, such as a round cylinder cut in the diameter direction, or in a semi-spherical arrangement, when the irradiation object 6 is a portion having a curved surface, such as a person's head.

In a semi-spherical arrangement, for example, as shown in Fig. 6, the irradiation case 5 is formed in a semi-spherical shape and a first irradiating unit 1 and a second irradiating unit 2 are provided on the inner side of the spherical shape, their respective radiation fronts 15 and 25 being arranged so as to face towards the center of the sphere.

In this case, the radiation fronts 15 and 25 are arranged in a semi-spherical shape having a smaller diameter than the irradiation case 5, concentrically with the irradiation case 5, and the radiation fronts 15 and 25 are made to contact the irradiation object 6, or the like, by positioning a person's head, or the like, on the central side, namely, the inner side of the semi-sphere. In other words, the irradiation case 5 has the shape of a cap which fits onto or covers a person's head, or the like, and the radiation fronts 15 and 25 can readily be made to contact a semi-spherical irradiation object 6, such as a person's head.

Therefore, it is possible to achieve contact with an irradiation object 6 having a curved surface, over a broader range than with a planar arrangement of radiation fronts 15 and 25, the number of light irradiation operations performed on an irradiation object 6 having a curved surface can be reduced, and a user can readily be made to use light irradiation in a more continued fashion.

It is desirable to arrange the irradiation case 5 above the back of a chair or a chair-shaped massage device, since this enables light irradiation to be carried out on a user seated in a chair, and the user can be made to maintain the same posture for the time period during which light irradiation is performed (for instance, 15 minutes).

Although the present invention has been described with reference to certain preferred embodiments, numerous modifications and variations can be made by those skilled in the art without departing from the true spirit and scope of this invention, namely claims.

## Claims

1. A hair growth device comprising:
a first light irradiator (1) configured to irradiate an invisible light of invisible wavelength range, for hair growth; and
a second light irradiator (2) configured to irradiate a visible light of visible wavelength range,
wherein the second light irradiator (2) is configured to direct the visible light to either inside of a light irradiation area of the light from the first light irradiator (1) or outer circumference of the light irradiation area.

2. The hair growth device of claim 1, wherein a main irradiation wavelength of the light irradiated from the first light irradiator (1) is included in a wavelength range between 780 nm and 2000 nm.

3. The hair growth device of claim 1 or 2, further comprising a controller (3) configured to vary at least one of brightness and color of the light irradiated from the second light irradiator (2) in response to an irradiation time of the light irradiated from the first light irradiator (1).

4. The hair growth device of claim 1 or 2, further comprising a controller (3) configured to change at least one of brightness and color of the light irradiated from the second light irradiator (2) when the first light irradiator (1) stops irradiating the invisible light.

5. The hair growth device of claim 1 or 2,
wherein the first light irradiator (1) comprises a plurality of radiation fronts (15) from each of which the invisible light is irradiated,
wherein the second light irradiator (2) comprises a plurality of radiation fronts (25) from each of which the visible light is irradiated,
wherein the plurality of the radiation fronts (15) of the first light irradiator (1) are arranged in an alternating relation to the plurality of the radiation fronts (25) of the second light (2).

6. The hair growth device of claim 1 or 2, wherein the second light irradiator (2) comprises a plurality of radiation fronts (25) arranged in an annular shape.

7. The hair growth device of claim 1 or 2, wherein the first light irradiator (1) comprises a plurality of radiation fronts (15) arranged in a semi-spherical shape, each of light irradiation directions from the radiation fronts (15) being arranged so as to face towards the center of the semi-spherical shape.
